(19) Europäisches Patentamt

European Patent Office

Office européen des brevets

(11) **EP 1 642 524 A1**

(12) **EUROPEAN PATENT APPLICATION**

(43) Date of publication:
**05.04.2006 Bulletin 2006/14**

(51) Int Cl.:
***A61B 5/00*** (2006.01)

(21) Application number: **04030921.3**

(22) Date of filing: **28.12.2004**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **29.09.2004 JP 2004283155**

(71) Applicant: **Hitachi, Ltd.**
**Tokyo 100-8280 (JP)**

(72) Inventors:
- **Cho, Ok-Kyung**
  **Hitachi Ltd,**
  **IPG**
  **Chiyoda-ku**
  **Tokyo 100-8220 (JP)**

- **Kim, Yoon-Ok**
  **Hitachi, Ltd,**
  **IPG**
  **Chiyoda-ku**
  **Tokyo 100-8220 (JP)**
- **Hattori, Hideharu**
  **Hitachi, Ltd,**
  **IPG**
  **Chiyoda-ku**
  **Tokyo 100-8220 (JP)**
- **Mitsumaki, Hiroshi**
  **Hitachi, Ltd,**
  **IPG**
  **Chiyoda-ku**
  **Tokyo 100-8220 (JP)**

(74) Representative: **Strehl Schübel-Hopf & Partner**
**Maximilianstrasse 54**
**80538 München (DE)**

(54) **Method and apparatus for measuring blood sugar levels**

(57) A method and apparatus for non-invasivel.y measuring blood sugar levels based on temperature measurement. A non-invasively determined blood sugar level measurement value is corrected on the basis of the blood oxygen saturation value and the blood flow volume, thereby stabilizing measurement data. A measurement portion is provided with a cover with which the measurement portion can be covered and protected when not in use. By controlling the opening and closing of the cover, erroneous measurement can be prevented. The intensity of a detection signal in an optical system is corrected by a circuit.

FIG. 5

**Description**

CLAIM OF PRIORITY

The present application claims priority from Japanese application JP 2004-283155 filed on September 29, 2004, the content of which is hereby incorporated by reference into this application.

CROSS REFERENCE TO RELATED APPLICATION

US Patent application No.10/620,689 is a co-pending application of this application. The content of which is incorporated herein by cross-reference.

BACKGROUND OF THE INVENTION

Field of the Invention

The present invention relates to a non-invasive blood sugar level measuring method and apparatus for measuring glucose concentrations in a living body without taking a blood sample.

Description of Related Art

Hilson *et al.* report facial and sublingual temperature changes in diabetics following intravenous glucose injection (Non-Patent Document 1). Scott *et al.* discuss the issue of diabetics and thermoregulation (Non-Patent Document 2). Based on the knowledge gained from such researches, Cho *et al.* suggest a method and apparatus for determining blood glucose concentration by temperature measurement without requiring the collection of a blood sample (Patent Documents 1 and 2).
Various other attempts have been made to determine glucose concentration without blood sampling. For example, a method has been suggested (Patent Document 3) whereby a measurement site is irradiated with near-infrared light of three wavelengths, and the intensity of transmitted light as well as the temperature of the living body is detected. A representative value of the second-order differentiated value of absorbance is then calculated, and the representative value is corrected in accordance with the difference between the living body temperature and a predetermined reference temperature. The blood sugar concentration corresponding to the thus corrected representative value is then determined. An apparatus is also provided (Patent Document 4) whereby a measurement site is heated or cooled while monitoring the living body temperature. The degree of attenuation of light based on light irradiation is measured at the moment of temperature change so that the glucose concentration responsible for the temperature-dependency of the degree of light attenuation can be measured. Further, an apparatus is reported (Patent Document 5) whereby an output ratio between reference light and transmitted light following the irradiation of the sample is taken, and then a glucose concentration is calculated in accordance with a linear expression of the logarithm of the output ratio and the living body temperature.
[Non-Patent Document 1] Diabete & Metabolisme, "Facial and sublingual temperature changes following intravenous glucose injection in diabetics" by R.M. Hilson and T.D.R. Hockaday, 1982, 8, 15-19
[Non-Patent Document 2] Can. J. Physiol. Pharmacol., "Diabetes mellitus and thermoregulation" by A.R. Scott, T. Bennett, I.A. MacDonald, 1987, 65, 1365-1376
[Patent Document 1] U.S. Patent No. 5,924,996
[Patent Document 2] U.S. Patent No. 5,795,305
[Patent Document 3] JP Patent Publication (Kokai) No. 2000-258343 A
[Patent Document 4] JP Patent Publication (Kokai) No. 10-33512 A (1998)
[Patent Document 5] JP Patent Publication (Kokai) No. 10-108857 A (1998)

SUMMARY OF THE INVENTION

Glucose (blood sugar) in blood is used for glucose oxidation reaction in cells to produce necessary energy for the maintenance of living bodies. In the basal metabolism state, in particular, most of the produced energy is converted into heat energy for the maintenance of body temperature. Thus, it can be expected that there is some relationship between blood glucose concentration and body temperature. However, as is evident from the way sicknesses cause fever, the body temperature also fluctuates due to factors other than blood glucose concentration. While methods have been proposed to determine blood glucose concentration by temperature measurement without blood sampling, they could hardly be considered sufficiently accurate.

It is an object of the invention to provide a method and apparatus for determining blood glucose concentration with high accuracy based on temperature data of a test subject without blood sampling.

Blood sugar is delivered to the cells throughout the human body via blood vessel systems, particularly the capillary blood vessels. In the human body, complex metabolic pathways exist. Glucose oxidation is a reaction in which, fundamentally, blood sugar reacts with oxygen to produce water, carbon dioxide, and energy. Oxygen herein refers to the oxygen delivered to the cells via blood. The oxygen supply is determined by the blood hemoglobin concentration, the hemoglobin oxygen saturation, and the volume of blood flow. On the other hand, the heat produced in the body by glucose oxidation is dissipated from the body by convection, heat radiation, conduction, and so on. On the assumption that the body temperature is determined by the balance between the amount of energy produced in the body by glucose burning, namely heat production, and heat dissipation such as mentioned above, the inventors set up the following model:

(1) The amount of heat production and the amount of heat dissipation are considered equal.
(2) The amount of heat production is a function of the blood glucose concentration and the oxygen supply.
(3) The oxygen supply is determined by the blood hemoglobin concentration, the blood hemoglobin oxygen saturation, and the volume of blood flow in the capillary blood vessels.
(4) The amount of heat dissipation is mainly determined by heat convection and heat radiation.

According to this model, we achieved the present invention after realizing that blood sugar levels can be accurately determined on the basis of the results of measuring the temperature of a body surface and parameters relating to the blood oxygen concentration and the blood flow volume. Parameters can be measured, e.g., from a part of the human body, such as the fingertip. Parameters relating to convection and radiation can be determined by measuring the temperature on the fingertip. Parameters relating to the blood hemoglobin concentration and the blood hemoglobin oxygen saturation can be determined by spectroscopically measuring blood hemoglobin and then finding the ratio between hemoglobin bound with oxygen and hemoglobin not bound with oxygen. A parameter relating to the volume of blood flow can be determined by measuring the amount of heat transfer from the skin. A measurement portion is provided with a cover so that the measurement portion can be covered and protected by closing the cover when the apparatus is not in use.

In the event that the cover is opened or closed by the user during measurement, or that the cover of the measurement portion was not correctly opened in the first place, accurate measurement data would not be obtained and it would be impossible to determine a blood glucose concentration accurately. Further, an optical-system detection signal intensity fluctuates depending on a change in the amount of light produced by a light source due to changes in ambient temperature, or on a change in the sensitivity of a photodiode. Such fluctuation affects the measured optical data and is a main factor for the deterioration of accuracy in calculating a blood sugar level, and therefore it must be corrected. By controlling the opening and closing of the cover to prevent erroneous measurement, and by correcting the optical-system detection signal intensity, it becomes possible to determine the accurate blood glucose concentration based on thermal and optical measurement data of a test subject without taking a blood sample.

In one example, the invention provides a blood sugar level measurement apparatus comprising:

a heat amount measurement portion for measuring a plurality of temperatures deriving from a body surface and obtaining information that is used for calculating the amount of convective heat transfer and the amount of radiation heat transfer, both of which are related to the dissipation of heat from said body surface;

an oxygen amount measurement portion for obtaining information relating to the blood oxygen amount, said oxygen amount measurement portion comprising a blood flow volume measurement portion for obtaining information relating to the blood flow volume, and an optical measurement portion for obtaining the hemoglobin concentration and the hemoglobin oxygen saturation in blood;

an openable and closable cover with which said optical measurement portion can be covered;

a cover open/close detection portion for detecting whether said cover is open or closed;

a memory portion in which a relationship between parameters associated with said plurality of temperatures individually and with said blood oxygen amount and blood sugar levels is stored;

a computation portion for converting a plurality of measurement values inputted from said heat amount measurement portion and said oxygen amount measurement portion into said parameters individually, and for computing a blood sugar level by applying said parameters to said relationship stored in said memory portion;

a display portion for displaying the result calculated by said computation portion; and

an optical sensor correction portion for correcting the output of said optical measurement portion, wherein:

said blood flow volume measurement portion comprises a body-surface contact portion, a first temperature detector disposed adjacent to said body-surface contact portion, a second temperature detector for detecting the temperature at a location spaced apart from said body-surface contact portion, and a heat-conducting member connecting said body-surface contact portion and said second temperature detector, and wherein:

said optical sensor correction portion comprises an optical correction check portion for calculating a correction value from the output of said optical measurement portion, and an optical correction portion for correcting the output of said optical measurement portion using the correction value calculated by said optical correction check portion.

In another example, the invention provides a blood sugar level measurement apparatus comprising:

an ambient temperature measurement portion for measuring ambient temperature;
a body-surface contact portion with which a body surface comes into contact;
an adjacent temperature detector disposed adjacent to said body-surface contact portion;
a radiation heat detector for measuring radiation heat from said body surface;
a heat conducting member disposed adjacent to said body-surface contact portion;
an indirect temperature detector disposed adjacent to said heat conducting member and at the same time spaced apart from said body-surface contact portion, for detecting the temperature at said location spaced apart from said body-surface contact portion;
an optical measurement portion comprising a light source for irradiating said body-surface contact portion with light of at least two different wavelengths, and a photodetector;
an openable and closable cover with which said optical measurement portion can be covered;
a cover open/close detection portion for detecting whether said cover is open or closed;
a computation portion comprising a conversion portion and a processing portion, said conversion portion converting the outputs from said adjacent temperature detector, said indirect temperature detector, said ambient temperature measurement portion, said radiation heat detector, and said photodetector, into individual parameters, and said processing portion storing a relationship between said parameters and blood sugar levels in advance and calculating a blood sugar level by applying said parameters to said relationship;
an optical sensor correction portion for correcting the output of said photodetector; and
a display portion for displaying the result outputted from said computation portion, wherein:

said optical sensor correction portion comprises an optical correction check portion for calculating a correction value based on the output of said photodetector, and an optical correction portion for correcting the output of said photodetector using the correction value calculated by said optical correction check portion.

In accordance with the invention, a blood sugar level measurement apparatus is provided that is capable of determining a blood sugar level in a non-invasive measurement but with the same level of accuracy as that attained in conventional invasive methods.

BRIEF DESCRIPTION OF THE DRAWINGS

Fig. 1 shows a model of heat transfer from a body surface to a block.
Fig. 2 shows a temporal change in measurement values of temperatures $T_1$ and $T_2$.
Fig. 3 shows an example of a measurement of a temporal change in temperature $T_3$.
Fig. 4 shows the relationships between measurement values provided by various sensors and parameters derived therefrom.
Fig. 5 shows an upper plan view of a non-invasive blood sugar level measurement apparatus according to the invention.
Fig. 6 shows a measurement portion in detail.
Fig. 7 shows a functional block diagram of the apparatus.
Fig. 8 shows an operating procedure of the apparatus.
Fig. 9 shows an optical correction check process.
Fig. 10 shows an optical member disposed on the cover in detail.
Fig. 11 shows a side view of the non-invasive blood sugar level measurement apparatus according to the invention.
Fig. 12 shows an example of an optical characteristics coefficient.
Fig. 13 shows an example of an initial value of the optical characteristics coefficient.
Fig. 14 shows an example of the relationship between diffuse reflectance, optical-system intensity, intercept, and slope.

Fig. 15 shows an example of thresholds of fluctuation amount of optical-system intensity, and the presence or absence of correction.

Fig. 16 shows a conceptual chart illustrating the flow of data processing in the apparatus.

Fig. 17 shows a chart plotting the glucose concentration values calculated according to the present invention and the glucose concentration values measured by the enzymatic electrode method.

## DETAILED DESCRIPTION OF THE PREFERRED EMBODIMENTS

The invention will now be described by way of preferred embodiments thereof with reference made to the drawings.

Initially, the above-mentioned model will be described in more specific terms. Regarding the amount of heat dissipation, convective heat transfer, which is one of the main causes of heat dissipation, is related to temperature difference between the ambient (room) temperature and the body-surface temperature. The amount of heat dissipation due to radiation, which is another main cause of dissipation, is proportional to the fourth power of the body-surface temperature according to the Stefan-Boltzmann law. Thus, it can be seen that the amount of heat dissipation from the human body is related to the room temperature and the body-surface temperature. On the other hand, the amount of oxygen supply, which is a major factor related to the amount of heat production, is expressed as the product of hemoglobin concentration, hemoglobin oxygen saturation, and blood flow volume.

The hemoglobin concentration can be measured from the absorbance at the wavelength (equal-absorbance wavelength) at which the molar absorbance coefficient of the oxyhemoglobin is equal to that of the reduced (deoxy-) hemoglobin. The hemoglobin oxygen saturation can be measured by measuring the absorbance at the equal-absorbance wavelength and the absorbance at at least one different wavelength at which the ratio between the molar absorbance coefficient of the oxyhemoglobin and that of the reduced (deoxy-) hemoglobin is known, and then solving simultaneous equations. Namely, the hemoglobin concentration and hemoglobin oxygen saturation can be obtained by conducting the measurement of absorbance at at least two wavelengths.

The rest is the blood flow volume, which can be measured by various methods. One example will be described below.

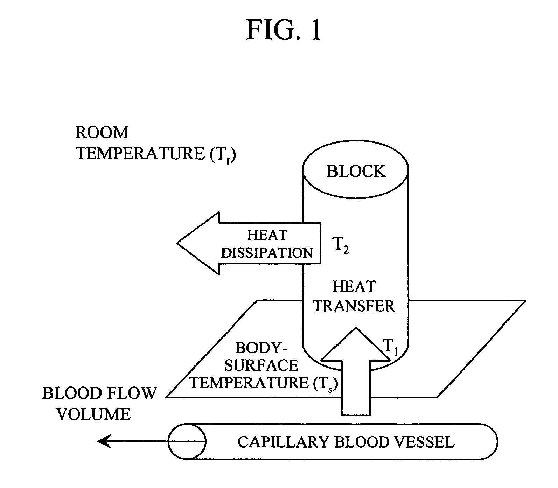

Fig. 1 shows a model for the description of the transfer of heat from a body surface to a solid block having a certain heat capacity when the block has been brought into contact with the body surface for a certain time and then separated. The block may be made of plastic or other resin, such as vinyl chloride. In the illustrated example, attention will be focused on the temporal variation of temperature $T_1$ of a portion of the block that is brought into contact with the body surface, and the temporal variation of temperature $T_2$ at a point on the block spaced apart from the body surface. The blood flow volume can be estimated by monitoring mainly the temporal variation of temperature $T_2$ (at the spatially separated point on the block). The details will follow.

Before the block comes into contact with the body surface, temperatures $T_1$ and $T_2$ at the two points of the block are equal to the room temperature $T_r$. When a body-surface temperature $T_s$ is higher than the room temperature $T_r$ as the block comes into contact with the body surface, temperature $T_1$ swiftly rises due to the transfer of heat from the skin, and it approaches the body-surface temperature $T_s$. On the other hand, temperature $T_2$ is attenuated relative to temperature $T_1$ as the heat conducted through the block is dissipated from the block surface, and it rises more gradually. The temporal variation of temperatures $T_1$ and $T_2$ depends on the amount of heat transferred from the body surface to the block, which in turn depends on the blood flow volume in the capillary blood vessels under the skin. If the capillary blood vessels are regarded as a heat exchanger, the coefficient of transfer of heat from the capillary blood vessels to the surrounding cell tissues is given as a function of the blood flow volume. Thus, by measuring the amount of heat transfer from the body surface to the block by monitoring the temporal variation of temperatures $T_1$ and $T_2$, the amount of heat transferred from the capillary blood vessels to the cell tissues can be estimated. Based on this estimation, the blood flow volume can then be estimated. Thus, by tracking the temporal temperature change in $T_1$ and $T_2$ and measuring the amount of heat transfer from the body surface to the block, the amount of heat transmitted from the capillary blood vessels to the cell tissue can be estimated, which in turn makes it possible to estimate the blood flow volume.

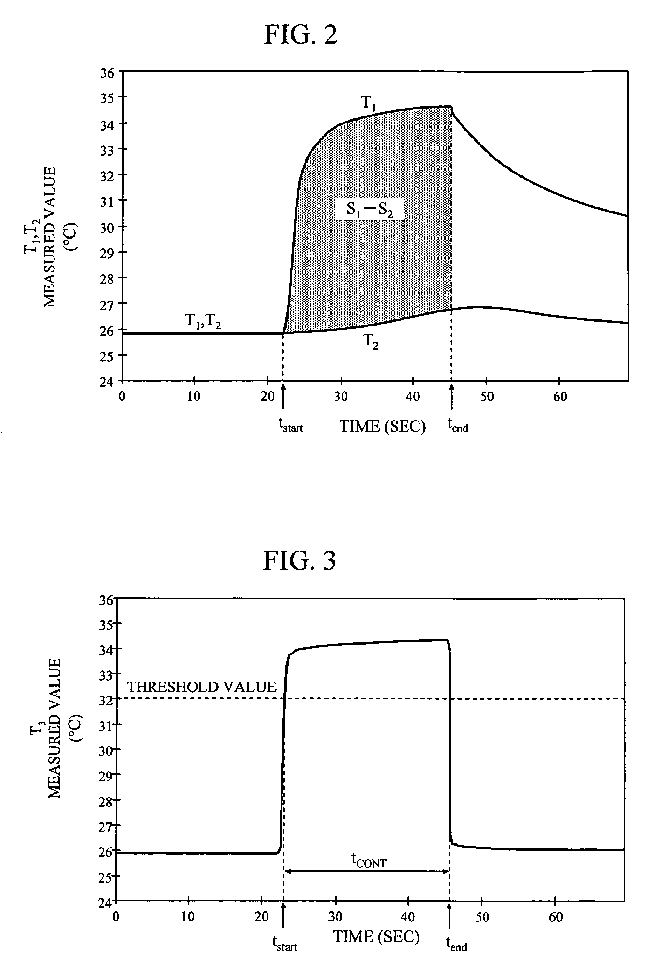

Fig. 2 shows the temporal variation of the measurement values of temperature $T_1$ at the portion of the block in contact with the body surface and temperature $T_2$ at the position on the block spaced apart from the body-surface contact position. As the block comes into contact with the body surface, the $T_1$ measurement value swiftly rises, and it gradually drops as the block is brought out of contact.

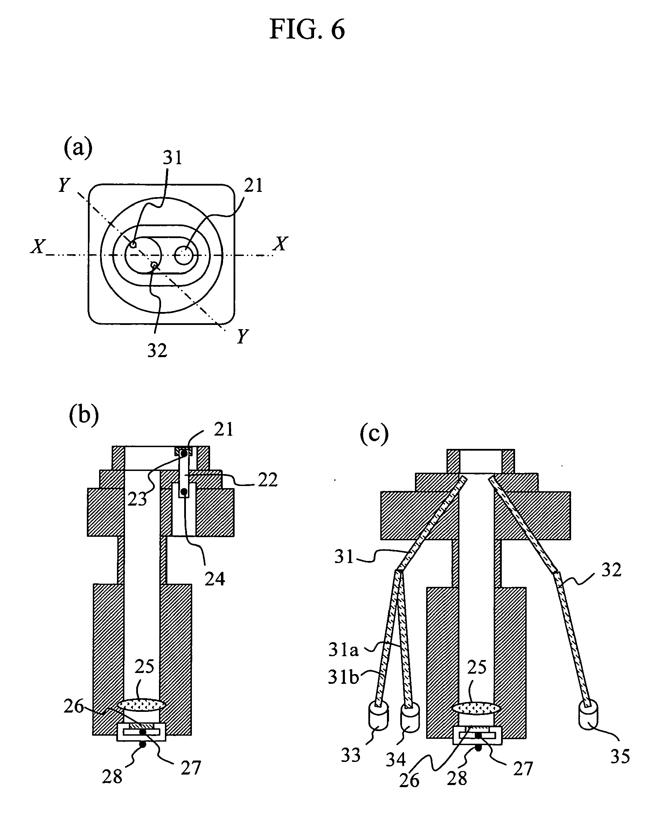

Fig. 3 shows the temporal variation of the value of temperature $T_3$ measured by a radiation-temperature detector. As the detector detects temperature $T_3$ that is due to radiation from the body surface, it is more sensitive to temperature changes than other sensors. Because radiation heat propagates as an electromagnetic wave, it can transmit temperature changes instantaneously. Thus, by locating the radiation-temperature detector near where the block contacts the body surface so as to detect radiated heat from the body surface, as shown in Fig. 6 (which will be described later), the time of start of contact $t_{start}$ and the time of end of contact tend between the block and the body surface can be detected from

changes in temperature $T_3$. For example, a temperature threshold value is set as shown in Fig. 3. The contact start time $t_{start}$ is when the temperature threshold value is exceeded. The contact end time $t_{end}$ is when temperature $T_3$ drops below the threshold. The temperature threshold is set at 32˚C, for example.

Then, the $T_1$ measurement value between $t_{start}$ and tend is approximated by an S curve, such as a logistic curve. A logistic curve is expressed by the following equation:

$$T = \frac{b}{1 + c \times \exp(-a \times t)} + d$$

where T is temperature, and t is time.

The measurement value can be approximated by determining coefficients a, b, c, and d using the non-linear least-squares method. For the resultant approximate expression, T is integrated between time $t_{start}$ and time $t_{end}$ to obtain a value $S_1$.

Similarly, an integrated value $S_2$ is calculated from the $T_2$ measurement value. The smaller ($S_1$ - $S_2$) is, the larger the amount of transfer of heat is from the finger surface to the position of $T_2$. ($S_1$ - $S_2$) becomes larger with increasing finger contact time $t_{CONT}$ (=$t_{end}$ - $t_{start}$). Thus, $a_5/(t_{CONT} \times (S_1 - S_2))$ is designated as a parameter $X_5$ indicating the volume of blood flow, using $a_5$ as a proportionality coefficient.

Thus, it will be seen that the measured amounts necessary for the determination of blood glucose concentration by the above-described model are the room temperature (ambient temperature), body surface temperature, temperature changes in the block brought into contact with the body surface, the temperature due to radiation from the body surface, and the absorbance of at least two wavelengths.

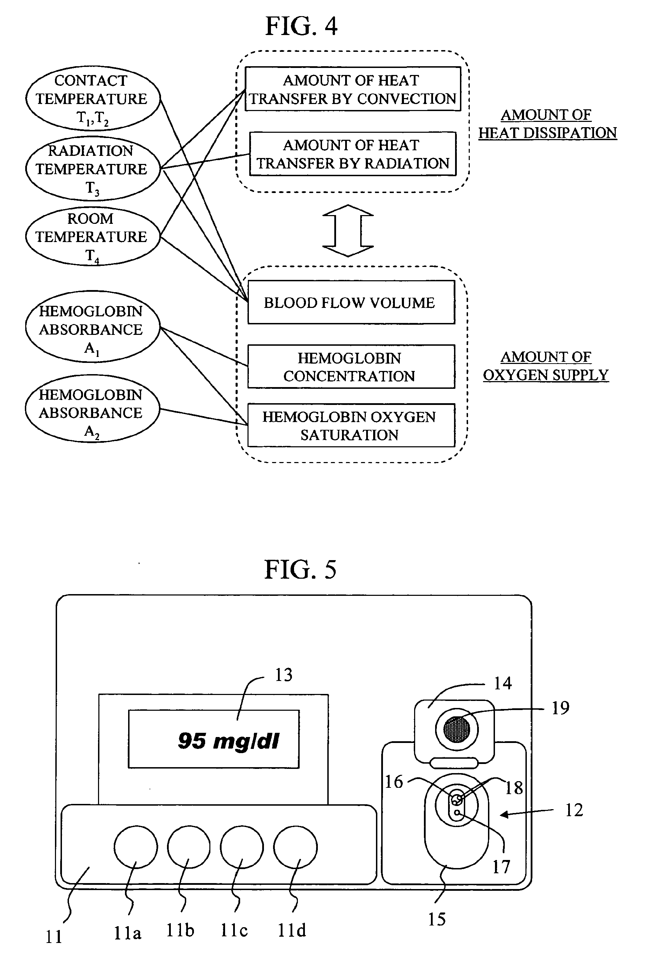

Fig. 4 shows the relationships between the measurement values provided by various sensors and parameters derived therefrom. A block is brought into contact with the body surface, and temporal changes in two kinds of temperatures $T_1$ and $T_2$ are measured by two temperature sensors provided at two locations on the block. Separately, radiation temperature $T_3$ on the body surface and room temperature $T_4$ are measured. Absorbance $A_1$ and $A_2$ are measured at at least two wavelengths related to the absorption of hemoglobin. Temperatures $T_1$, $T_2$, $T_3$, and $T_4$ provide parameters related to the volume of blood flow. Temperature $T_3$ provides a parameter related to the amount of heat transferred by radiation. Temperatures $T_3$ and $T_4$ provide parameters related to the amount of heat transferred by convection. Absorbance $A_1$ provides a parameter related to the hemoglobin concentration, and absorbance $A_1$ and $A_2$ provide a parameter related to the hemoglobin oxygen saturation.

Hereafter, an example of an apparatus for non-invasively measuring blood sugar levels according to the principle of the invention will be described.

Fig. 5 shows a top plan view of a non-invasive blood sugar level measurement apparatus according to the invention. While in this example the skin on the ball of the fingertip is used as the body surface, other parts of the body surface may be used.

On the top surface of the apparatus are provided an operating portion 11, a measurement portion 12 where the finger to be measured is to be placed, and a display portion 13 for displaying measurement results, the state of the apparatus, measurement values, for example. The operating portion 11 includes four push buttons 11a to 11d for operating the apparatus. The measurement portion 12 has a cover 14 which, when opened (as shown), reveals a finger rest portion 15 with an oval periphery. The finger rest portion 15 accommodates an opening end 16 of a radiation-temperature sensor portion, a contact-temperature sensor portion 17, and an optical sensor portion 18.

Fig. 6 shows the measurement portion in detail. In Fig. 6, (a) is a top plan view, (b) is a cross section taken along line X-X of (a), and (c) is a cross section taken along line Y-Y of (a).

First, the process of measuring temperatures by the non-invasive blood sugar level measurement apparatus according to the invention will be described. On a part with which the examined portion (ball of the finger) is to come into contact, there is placed a thin plate 21 of a highly heat-conductive material, such as gold. A bar-shaped heat-conductive member 22, which is made of a material with a heat conductivity lower than that of the plate 21, such as polyvinylchloride, is thermally connected to the plate 21 and extends into the apparatus. The temperature sensors include a thermistor 23 that is an adjacent temperature detector with respect to the examined portion for measuring the temperature of the plate 21, and a thermistor 24 that is an indirect temperature detector with respect to the examined portion for measuring the temperature of a portion of the heat-conducting member which is spaced apart from the plate 21 by a certain distance. An infrared lens 25 is disposed inside the apparatus at such a position that the examined portion (ball of the finger) placed on the finger rest portion 15 can be seen through the lens. Below the infrared lens 25 is disposed a pyroelectric detector 27 via an infrared radiation-transmitting window 26. Another thermistor 28 is disposed in close proximity to the pyroelectric detector 27.

Thus, the temperature sensor portion of the measurement portion has four temperature sensors, and they measure four kinds of temperatures as follows:

(1) Temperature on the finger surface (thermistor 23): $T_1$
(2) Temperature of the heat-conducting member (thermistor 24): $T_2$
(3) Temperature of radiation from the finger (pyroelectric detector 27): $T_3$
(4) Room temperature (thermistor 28): $T_4$

The optical sensor portion 18 is described hereafter. The optical sensor portion 18 measures the hemoglobin concentration and the hemoglobin oxygen saturation necessary for the determination of the oxygen supply. In order to measure the hemoglobin concentration and the hemoglobin oxygen saturation, it is necessary to measure absorbance at at least two wavelengths. Figs. 6(c) shows an example for carrying out a two-wavelength measurement using two light sources 33 and 34 and a single detector 35.

The ends of two optical fibers 31 and 32 are located in the optical sensor portion 18. The optical fiber 31 is for optical irradiation, while the optical fiber 32 is for receiving light. As shown in Fig. 6(c), the optical fiber 31 connects to branch optical fibers 31a and 31b. Light-emitting diodes 33 and 34 of two wavelengths are provided at the ends of the branch optical fibers 31a and 31b, respectively. The other end of the light-receiving optical fiber 32 is provided with a photodiode 35. The light-emitting diode 33 emits light with a wavelength of 810 nm, while the light-emitting diode 34 emits light with a wavelength of 950 nm. The wavelength 810 nm is the equal-absorbance wavelength at which the molar absorbance coefficient of the oxyhemoglobin is equal to that of the reduced (deoxy-) hemoglobin. The wavelength 950 nm is the wavelength at which the difference between the molar absorbance coefficient of the oxyhemoglobin and that of the reduced hemoglobin is large.

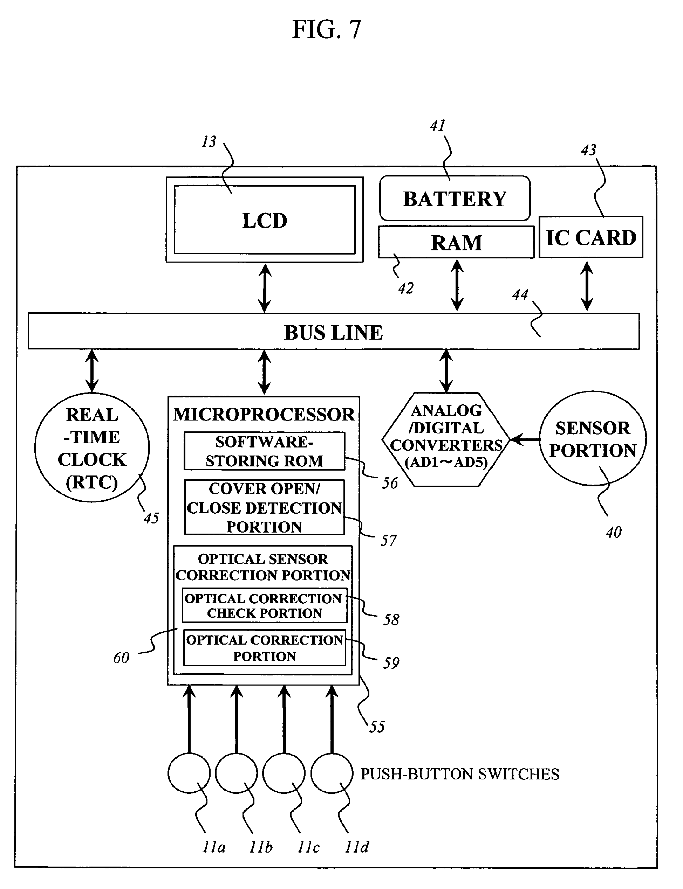

Fig. 7 shows a functional block diagram of the apparatus, which is operated by a battery 41. Peripheral circuits to a microprocessor 55 include analog/digital converters AD1 to AD5, an LCD 13, a RAM 42, an IC card 43, and a real-time clock 45. These circuits are accessed by the microprocessor 55 via a bus line 44. Push buttons 11a to 11d are individually connected to the microprocessor 55.

Signals measured by a sensor portion 40 made up of the temperature and optical sensors are fed to analog/digital converters AD1 to AD5 provided for corresponding signals, where the signals are converted into digital signals. The microprocessor 55 includes a ROM 56 for storing software, a cover open/close detection portion 57, an optical correction check portion 58, and an optical correction portion 59. The optical correction check portion 58 and the optical correction portion 59 constitute an optical sensor correction portion 60. The cover open/close detection portion 57 detects the state of the cover, i.e., whether it is open or closed, and causes a relevant message to be displayed on the LCD 13 as needed. The optical correction check portion 58 measures the optical-system intensity using the sensor portion 40 and calculates an optical characteristics coefficient. The optical correction check portion 58 then determines a fluctuation amount from the thus calculated optical characteristics coefficient and its initial value that is read from the IC card 43. The optical correction check portion 58 then performs a threshold determination regarding the fluctuation amount, and, if correction is necessary, calculates a correction value and stores it in RAM 42. In the event that a correction value is stored in RAM 42 by the optical correction check portion 58, the optical correction portion 59 reads the correction value from RAM 42 and calculates a corrected measurement value based on a measurement value.

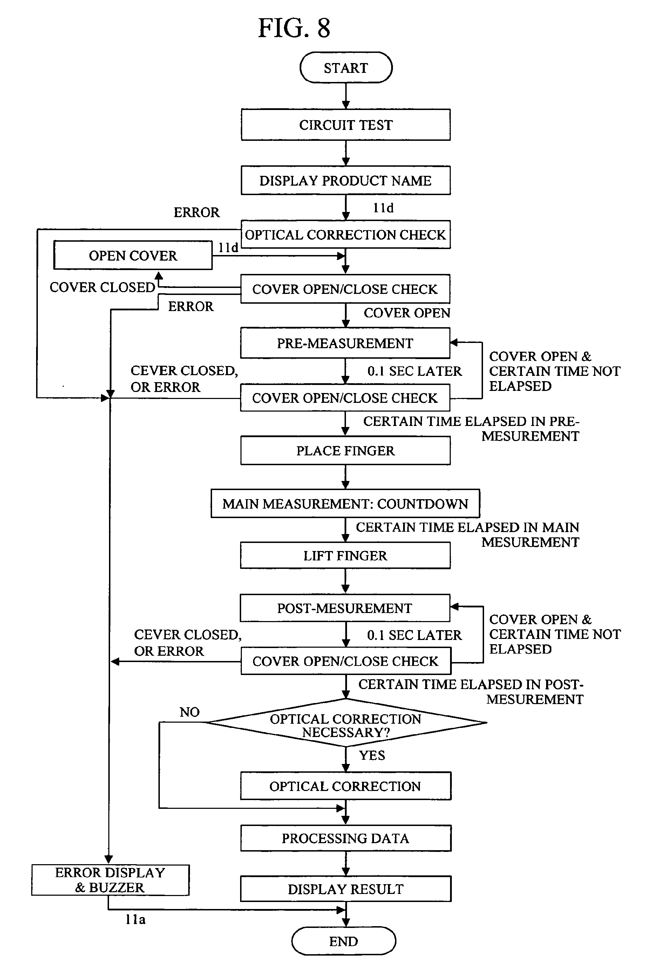

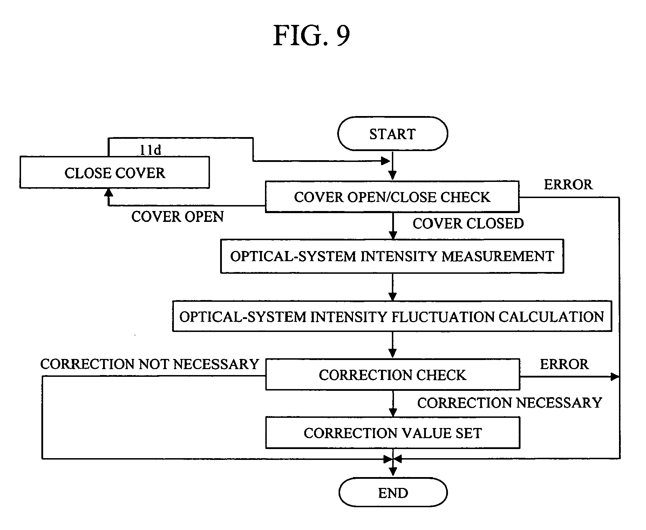

Fig. 8 shows an operating procedure of the apparatus. As a particular button in the operating portion is pressed to turn on the apparatus, a check program is activated whereby the electric circuits are automatically checked. Thereafter, the product name is displayed on the LCD and the apparatus awaits for key entry. Prior to measurement, button 11d is pressed to perform an optical correction check. Fig. 9 shows a flowchart of the optical correction check procedure. In a cover opening/closing check process, an optical member 19 disposed on the inside of a cover 14 is irradiated with light when the cover 14 is at a location with respect to closing motion. The optical member 19 may be made of silicon rubber and have a reflectance of 70%, for example. A resultant measurement value based on the output of a photodetector is compared with a reference value that is stored in a memory, such as IC card 43, in advance, in order to confirm that the cover is closed.

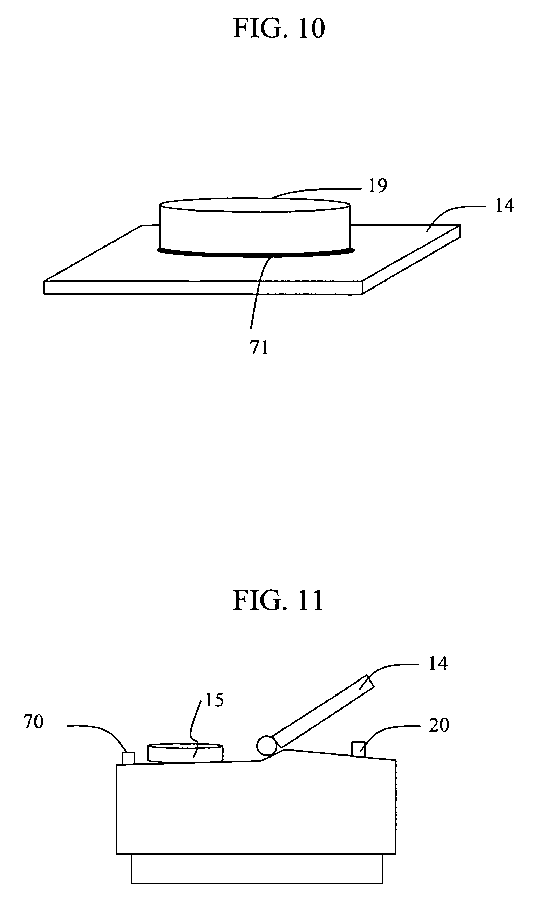

With reference to Fig. 10, a paint 71, such as black-body paint, is applied to the back surface of the optical member 19 via which the cover 14 is in contact with the optical member 19. The paint 71 thus applied absorbs light that has passed through the optical member 19, thereby preventing the reflection of light on the back surface and maintaining a uniform reflectance on the surface of the optical member 19. Using an optical member 19 such as the one mentioned above allows the optical system intensity to be accurately measured.

Further, when the cover 14 is at a location with respect to closing motion, a measurement value (base value) based on the output of the photodetector without irradiating the optical member 19 disposed on the inside of the cover 14 with light is compared with a reference base value that is stored in a memory such as IC card 43 in advance, so as to confirm that the cover is open. More specifically, the open or closed state of the cover is determined based on the following conditions. It is noted, however, that condition (2) described below may be substituted by condition (3) if the measurement is made in a rather dark room without illumination. It is also possible to use condition (4) instead of condition (1).

(1) The cover is determined to be closed if reference value × 0.9 ≤ measurement value ≤ reference value × 1.1.
(2) The cover is determined to be open if condition (1) is not satisfied and if reference base value × 1.1 < measurement (base) value.
(3) A hard switch 20 for detecting the open state of the cover is provided (such that as the cover 14 is opened and rotated, the switch 20 is depressed), as shown in Fig. 11. The open state of the cover is detected upon detection of depressing of the hard switch 20.
(4) A hard switch 70 for detecting the closed state of the cover is provided (such that as the cover is closed, the switch 70 is depressed), as shown in Fig. 11. The closed state of the cover is detected upon detection of depressing of the hard switch 70.
(5) If none of the above conditions (1) to (4) is satisfied, an error is presumed.

When the cover is closed, the intensity at each wavelength is measured in the subsequent optical-system intensity measurement process. If the cover is not closed, a message "Close the cover" is shown on the LCD, prompting the user to close the cover. When there is the message "Close the cover" on the LCD, if the user presses the button 11d after closing the cover, the optical-system intensity measurement process is performed.

In the optical-system intensity measurement process, an intensity measurement (normal measurement) in the case where the light sources 33 and 34 emitted light and an intensity measurement (offset measurement) in the case where the light sources 33 and 34 did not emit light are performed. Based on the result of the optical-system intensity measurement process, an average value and an offset value of measurement values are calculated depending on combinations of a light source and a photodetector, respectively.

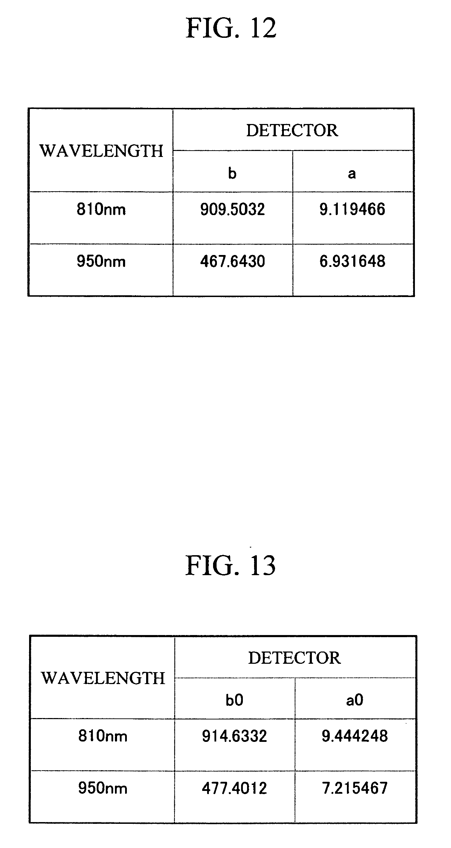

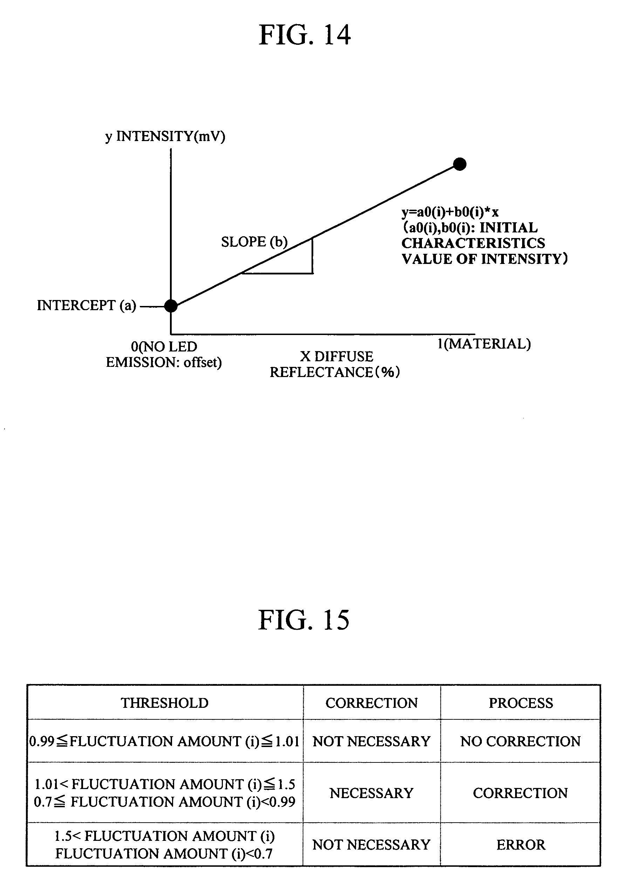

Thereafter, a fluctuation amount of the optical-system intensity is calculated in an optical-system intensity fluctuation calculation process. In this process, an optical characteristics coefficient is calculated from the average value and offset value calculated in the optical-system intensity measurement process, as shown in an example of Fig. 12. The optical characteristics coefficient consists of the slope (b) and the intercept (a) of a measurement value calculated from measurement data, with the diffuse reflectance of the optical member 19 normalized as "1." With reference to an example shown in Fig. 13, the slope (b0) and the intercept (a0) of an initial value of the optical characteristics coefficient that have been calculated in advance based on a measurement using the optical member 19 are stored in a memory, such as the IC card 43. Fig. 14 shows the relationship between diffuse reflectance, optical-system intensity, intercept, and slope. The optical-system intensity is a linear equation with respect to diffuse reflectance, intercept, and slope. Based on the aforementioned initial value and the optical characteristics coefficient of measurement values, a fluctuation amount is calculated according to the following equation:

$$\text{Fluctuation amount (i)} = \text{measurement value } (a(i) + b(i))/\text{initial value}$$

$$(a0(i) + b0(i))$$

where i = 1 to 2.

In the examples of Figs. 12 and 13, the fluctuation amount calculated for each wavelength is as follows:
Fluctuation amount for 810 nm (1) = 0.9940970
Fluctuation amount for 950 nm (2) = 0.9792784

Thereafter, in a correction check process, a threshold determination is performed on each of the thus calculated fluctuation values so as to determine whether or not a correction is required and to select a process to be performed. Fig. 15 shows examples of the range of threshold and the process content. If the fluctuation amount in the optical-system intensity is such that 0.99 ≤ fluctuation amount (i) ≤ 1.01, no correction is required, so that a correction-value setting process is not performed. If the fluctuation amount of the optical-system intensity is such that 1.01 < fluctuation amount (i) ≤ 1.5, or 0.7 ≤ fluctuation amount (i) < 0.99, a correction is required, so that the subsequent correction-value setting process is performed. If the fluctuation amount of the optical-system intensity is such that 1.5 < fluctuation amount (i), or fluctuation amount (i) < 0.7, the correction range is exceeded, so that the subsequent correction-value setting process is not performed but an error is indicated on the LCD before the measurement comes to an end.

In the examples of Figs. 12 and 13, since fluctuation amount (1) for 810 nm is such that 0.99 ≤ fluctuation amount (1) ≤ 1.01, the correction-value setting process is not performed. On the other hand, since fluctuation (2) for 950 nm is such that 0.7 ≤ fluctuation amount (2) < 0.99, the correction-value setting process is performed.

In the correction-value setting process, a correction value that is used for correcting the optical-system intensity is calculated and stored in a memory such as RAM 42, for example. The correction value is calculated according to the following equations:

$$\text{Correction value G: } G(i) = b0(i)/b(i)$$

$$\text{Correction value O: } O(i) = a0(i) - b0(i) \times a(i)/b(i)$$

In the examples of Figs. 12 and 13, correction values G and O for 950 nm are calculated as follows:

$$\text{Correction value } G(2) = 1.0208667723$$

$$\text{Correction value } O(2) = 0.1391778795$$

Reference is now made to Fig. 8 again. Because a correct measurement following the optical correction check process requires the cover 14 of the measurement portion to be open, a cover open/close check process is performed. If the cover is closed, a message "Open the cover" is shown on the LCD, prompting the user to open the cover. Once it is confirmed that the cover is open, a measurement is made in a pre-measurement process at 0.1 second intervals, for a certain duration of time, thereby acquiring data. During each of the 0.1 second intervals, the cover open/close check process is carried out while acquiring data. If it is confirmed that the cover is closed, the measurement is terminated and an error is indicated on the LCD. If the pre-measurement process comes to an end after the certain duration of time without the cover 14 being closed, a message "Place finger" is shown on the LCD. When the finger is placed on the finger rest portion 15, a main measurement process is initiated while displaying a countdown on the LCD. After a certain time elapses and the main measurement process is completed with the end of the countdown, a message "Lift finger" is displayed on the LCD. As the user lifts his or her finger from the finger rest portion 15, a measurement is made in a post-measurement process at 0.1 second intervals for a certain duration of time, thereby acquiring data. During each of the 0.1 second intervals, the cover open/close check process is also performed while acquiring data. If the cover is closed during measurement, the measurement is terminated and an error is indicated. If the post-measurement process is completed after the certain duration of time without the cover 14 being closed, an optical correction process is subsequently performed.

In the case where a correction value has been set in the correction-value setting process in the optical correction check process, an optical correction process is performed. The correction values G and O calculated in the correction-value setting process are read from RAM 42, and corrected measurement values are obtained based on an actually measured value, in accordance with the following equation:

$$\text{Corrected measurement value } (i) = O(i) + G(i) \times \text{measurement value } (i)$$

In the examples of Figs. 12 and 13, if measurement value (2) is 474.574648 mV, for example, the corrected measurement value would be as follows:

$$\text{Corrected measurement value } (2) = 484.616667 \text{ mV}$$

After the optical correction process is completed, a message "Processing data" is displayed on the LCD while data processing is performed using the value of the corrected measurement value. In the case where a correction value has not been set in the correction-value setting process in the optical correction check process, the optical correction process is not performed. In this case, the message "Processing data" is displayed on the LCD and data processing is performed using the measurement value that has not been corrected.

At the end of the data processing, a blood sugar level is displayed on the LCD. At this point in time, the blood sugar level being displayed is stored in the IC card, together with date and time. After the user has read the displayed blood sugar level, if he or she presses the button 11d in the operating portion, the apparatus initiates the optical correction check process for the next measurement approximately one minute later.

By thus performing the cover open/close check process, it becomes possible to prevent erroneous measurement and acquire highly accurate temperature and optical measurement data. Further, by performing the optical correction check process and the optical correction process, the optical-system intensity can be stabilized during measurement, so that stable data can be obtained over a plurality of measurements and the accuracy of the calculated blood sugar level can be improved.

Referring back to Fig. 6, the operation of the measurement portion during measurement will be described. The two light-emitting diodes 33 and 34 emits light in a time-shared manner. The light emitted by the light-emitting diodes 33 and 34 is shone on the finger of the subject via the light-irradiating optical fiber 31. The light shone on the finger is reflected by the skin of the finger and is then incident on the light-receiving optical fiber 32, before it is eventually detected by the photodiode 35. When the light with which the skin of the finger is irradiated is reflected by the skin, some of the light penetrates into the tissue via the skin and is absorbed by the hemoglobin in the blood that flows in the capillary blood vessels. The photodiode 35 provides data that is reflectance R, and absorbance is approximately calculated by $\log(I/R)$. Irradiation is performed at wavelengths of 810 nm and 950 nm, R is measured for each wavelength, and $\log(1/R)$ is determined for each. In this way, absorbance $A_1$ for wavelength 810 nm and absorbance $A_2$ for wavelength 950 nm are measured.

When the reduced hemoglobin concentration is [Hb] and the oxyhemoglobin concentration is $[HbO_2]$, absorbance $A_1$ and absorbance $A_2$ are expressed by the following equations:

$$A_1 = a \times ([Hb] \times A_{Hb}(810nm) + [HbO_2] \times A_{HbO_2}(810nm))$$

$$= a \times ([Hb] + [HbO_2]) \times A_{HbO_2}(810nm)$$

$$A_2 = a \times ([Hb] \times A_{Hb}(950nm) + [HbO_2] \times A_{HbO_2}(950nm))$$

$$= a \times ([Hb] + [HbO_2]) \times ((1 - \frac{[HbO_2]}{[Hb] + [HbO_2]}) \times A_{Hb}(950nm) + \frac{[HbO_2]}{[Hb] + [HbO_2]} \times A_{HbO_2}(950nm))$$

$A_{Hb}$(810 nm) and $A_{Hb}$(950 nm), and $A_{Hb02}$ (810 nm) and $A_{Hb02}$ (950 nm) are the molar absorption coefficients of the reduced hemoglobin and the oxyhemoglobin, respectively, and are known at each wavelength. Term a is a proportionality coefficient. From the above equations, the hemoglobin concentration $[Hb]+[HbO_2]$ and the hemoglobin oxygen saturation $[HbO_2]/([Hb]+[HbO_2])$ are determined as follows:

$$[Hb] + [HbO_2] = \frac{A_1}{a \times A_{HbO_2}(810nm)}$$

$$\frac{[HbO_2]}{[Hb] + [HbO_2]} = \frac{A_2 \times A_{HbO_2}(810nm) - A_1 \times A_{Hb}(950nm))}{A_1 \times (A_{HbO_2}(950nm) - A_{Hb}(950nm))}$$

Although in the above-described example the hemoglobin concentration and the hemoglobin oxygen saturation have been measured by measuring absorbance at two wavelengths, it is also possible to reduce the influence of interfering components and thereby enhance measurement accuracy by measuring absorbance at three or more wavelengths.

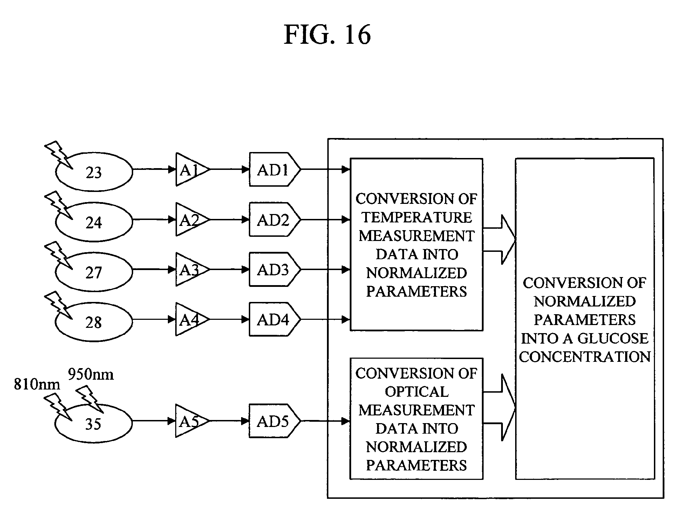

Fig. 16 is a conceptual chart showing the flow of data processing in the apparatus. The apparatus according to the present example is equipped with a thermistor 23, a thermistor 24, a pyroelectric detector 27, a thermistor 28, and a photodiode 35, for a total of five sensors. The photodiode 35 measures absorbance at wavelengths 810 nm and 950 nm, so that the apparatus is supplied with six kinds of measurement values.

The five kinds of analog signals are supplied via individual amplifiers A1 to A5 to analog/digital converters AD1 to AD5, where they are converted into digital signals. Based on the digitally converted values, parameters $x_i$ (i=1, 2, 3, 4, 5) are calculated. The following are specific descriptions of $x_i$ (where $a_1$ to $a_5$ are proportionality coefficients):

Parameter proportional to heat radiation

$$x_1 = a_1 \times (T_3)^4$$

Parameter proportional to heat convection

$$x_2 = a_2 \times (T_4 - T_3)$$

Parameter proportional to hemoglobin concentration

$$x_3 = a_3 \times \left( \frac{A_1}{a \times A_{HbO_2}(810nm)} \right)$$

Parameter proportional to hemoglobin oxygen saturation

$$x_4 = a_4 \times \left( \frac{A_2 \times A_{HbO_2}(810nm) - A_1 \times A_{Hb}(950nm)}{A_1 \times (A_{HbO_2}(950nm) - A_{Hb}(950nm))} \right)$$

Parameter proportional to blood flow volume

$$x_5 = a_5 \times \left( \frac{1}{t_{CONT} \times (S_1 - S_2)} \right)$$

Then, normalized parameters are calculated from mean values and standard deviations of parameter $x_i$ obtained from actual data pertaining to large numbers of able-bodied people and diabetic patients. A normalized parameter $X_i$ (where i=1, 2, 3, 4, 5) is calculated from each parameter $x_i$ according to the following equation:

$$X_i = \frac{x_i - \overline{x_i}}{SD\ (x_i)}$$

where
$x_i$: parameter
$\overline{x}_i$ : mean value of the parameter
$SD(x_i)$: standard deviation of the parameter
Using the above five normalized parameters, calculations are conducted for conversion into a glucose concentration that is eventually displayed. A program necessary for the calculations is stored in a ROM in the microprocessor built inside the apparatus. The memory area required for the calculations is secured in a RAM similarly built inside the apparatus. The result of calculation is displayed on the LCD.
The ROM stores, as a constituent element of the program necessary for the calculations, a function for determining glucose concentration C in particular. The function is defined as follows. C is expressed by the below-indicated equation (1), where $a_i$ (i=0, 1, 2, 3, 4, 5) is determined from a plurality of pieces of measurement data in advance according to the following procedure:

(1) A multiple regression equation is created that indicates the relationship between the normalized parameters and glucose concentration C.

(2) Normalized equations (simultaneous equations) relating to the normalized parameters are obtained from equations obtained by the least-squares method.

(3) Values of coefficient $a_i$ (i=0, 1, 2, 3, 4, 5) are determined from the normalized equations and then substituted into the multiple regression equation.

Initially, the regression equation (1) indicating the relationship between glucose concentration C and the normalized parameters $X_1$, $X_2$, $X_3$, $X_4$, and $X_5$ is formulated.

$$C = f(X_1, X_2, X_3, X_4, X_5)$$
$$= a_0 + a_1 X_1 + a_2 X_2 + a_3 X_3 + a_4 X_4 + a_5 X_5 \quad ......(1)$$

Then, the least-squares method is employed to obtain a multiple regression equation that would minimize the error with respect to a measurement value $C_i$ of glucose concentration according to an enzyme electrode method. When the sum of squares of the residual is D, D is expressed by the following equation (2):

$$D = \sum_{i=1}^{n} d_i^2$$
$$= \sum_{i=1}^{n} (C_i - f(X_{i1}, X_{i2}, X_{i3}, X_{i4}, X_{i5}))^2$$
$$= \sum_{i=1}^{n} \{C_i - (a_0 + a_1 X_{i1} + a_2 X_{i2} + a_3 X_{i3} + a_4 X_{i4} + a_5 X_{i5})\}^2 \quad ......(2)$$

The sum D of the squares of the residual becomes minimum when partial differentiation of equation (2) with respect to $a_0$, $a_2$,..., $a_5$ gives zero. Thus, we have the following equations:

$$\frac{\partial D}{\partial a_0} = -2 \sum_{i=1}^{n} \{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_1} = -2 \sum_{i=1}^{n} X_{i1} \{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_2} = -2 \sum_{i=1}^{n} X_{i2} \{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_3} = -2 \sum_{i=1}^{n} X_{i3} \{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_4} = -2 \sum_{i=1}^{n} X_{i4} \{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_5} = -2 \sum_{i=1}^{n} X_{i5} \{C_i - (a_0 + a_1 X_{i1} + a_2 X_{i2} + a_3 X_{i3} + a_4 X_{i4} + a_5 X_{i5})\} = 0 \quad ......(3)$$

When the mean values of C and $X_1$ to $X_5$ are $C_{mean}$ and $X_{1mean}$ to $X_{5mean}$, respectively, since $X_{imean}=0$ (i=1 to 5), equation (1) yields:

$$a_0 = C_{mean} - a_1 X_{1mean} - a_2 X_{2mean} - a_3 X_{3mean} - a_4 X_{4mean} - a_5 X_{5mean}$$
$$= C_{mean} \qquad \qquad \cdots\cdots(4)$$

The variation and covariation between the normalized parameters are expressed by equation (5). Covariation between the normalized parameter $X_i$ (i=1 to 5) and C is expressed by equation (6).

$$S_{ij} = \sum_{k=1}^{n}(X_{ki} - X_{imean})(X_{kj} - X_{jmean}) = \sum_{k=1}^{n} X_{ki} X_{kj} \quad (i,j = 1,2,..5) \quad \cdots\cdots(5)$$

$$S_{iC} = \sum_{k=1}^{n}(X_{ki} - X_{imean})(C_k - C_{mean}) = \sum_{k=1}^{n} X_{ki}(C_k - C_{mean}) \quad (i = 1,2,..5) \quad \cdots\cdots(6)$$

Substituting equations (4), (5), and (6) into equation (3) and rearranging yields a set of simultaneous equations (normalized equations) (7). Solving the set of equations (7) yields $a_1$ to $a_5$.

$$a_1 S_{11} + a_2 S_{12} + a_3 S_{13} + a_4 S_{14} + a_5 S_{15} = S_{1C}$$
$$a_1 S_{21} + a_2 S_{22} + a_3 S_{23} + a_4 S_{24} + a_5 S_{25} = S_{2C}$$
$$a_1 S_{31} + a_2 S_{32} + a_3 S_{33} + a_4 S_{34} + a_5 S_{35} = S_{3C}$$
$$a_1 S_{41} + a_2 S_{42} + a_3 S_{43} + a_4 S_{44} + a_5 S_{45} = S_{4C}$$
$$a_1 S_{51} + a_2 S_{52} + a_3 S_{53} + a_4 S_{54} + a_5 S_{55} = S_{5C} \quad \cdots\cdots(7)$$

Constant term $a_0$ is obtained by means of equation (4). The thus obtained $a_i$ (i=0, 1, 2, 3, 4, 5) is stored in ROM at the time of manufacture of the apparatus. In an actual measurement using the apparatus, the normalized parameters $X_1$ to $X_5$ obtained from the measurement values are substituted into regression equation (1) to calculate glucose concentration C.

Hereafter, an example of the process of calculating the glucose concentration will be described. The coefficients for regression equation (1) are determined in advance based on many items of data obtained from able-bodied persons and diabetics, and the ROM in the microprocessor stores the following formula for calculating the glucose concentration:

$$C = 99.4 + 18.3 \times X_1 - 20.2 \times X_2 - 23.7 \times X_3 - 22.0 \times X_4 - 25.9 \times X_5$$

$X_1$ to $X_5$ are the results of normalization of the above-obtained parameters $x_1$ to $x_5$. Assuming the distribution of a parameter is normal, 95% of a normalized parameter takes on values between -2 and +2.

In an example of measurement values for an able-bodied person, substituting normalized parameters $X_1 = -0.06$, $X_2 = +0.04$, $X_3 = +0.05$, $X_4 = -0.12$, and $X_5 = +0.10$ into the above equation gives C=96 mg/dl. In an example of measurement values for a diabetic patient, substituting normalized parameters $X_1 = +1.15$, $X_2 = -1.02$, $X_3 = -0.83$, $X_4 = -0.91$, and $X_5 = -1.24$ into the equation yields C=213 mg/dl.

The following describes the results of measurement by the conventional enzymatic electrode method in which a blood sample is reacted with a reagent and the amount of resultant electrons is measured to determine glucose concentration, and the results of measurement by an embodiment of the invention. When the glucose concentration for an able-bodied person was 89 mg/dl according to the enzymatic electrode method in one example, substituting the normalized parameters $X_1 = -0.06$, $X_2 = +0.04$, $X_3 = +0.05$, $X_4 = -0.12$, and $X_5 = +0.10$, which were obtained by measurement at the same time according to the invention, into the above equation yields C=96 mg/dl. In another example, when the measurement value of glucose concentration for a diabetic patient was 238 mg/dl according to the enzymatic electrode method, substituting the normalized parameters $X_1 = +1.15$, $X_2 = -1.02$, $X_3 = -0.83$, $X_4 = -0.91$, and $X_5 = -1.24$, which were obtained by measurement at the same time according to the invention, into the above equation yields C=213 mg/dl. The results thus indicated that the method according to the invention can provide highly accurate glucose concentration values.

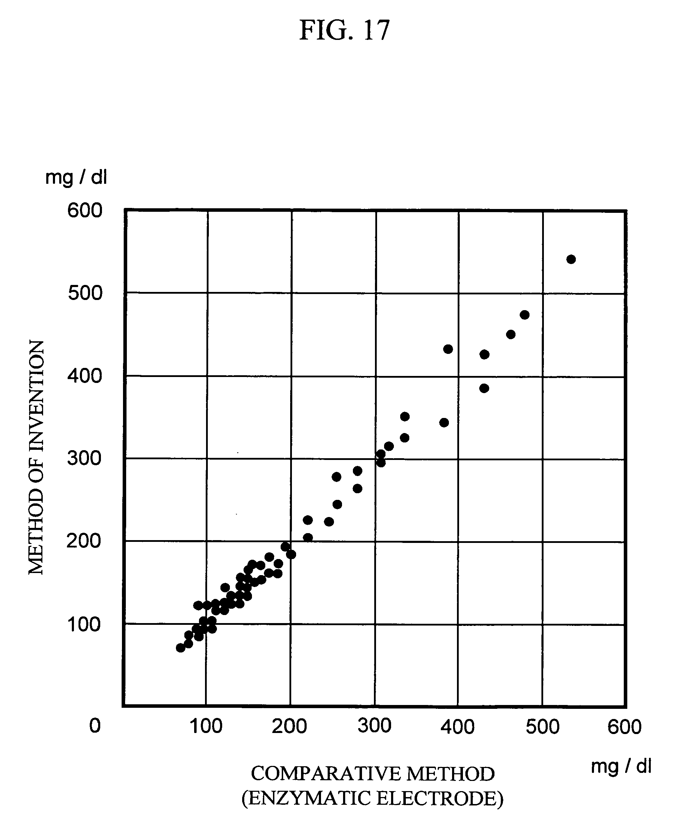

Fig. 17 shows a graph plotting glucose concentrations for a plurality of patients, with the vertical axis showing the calculated values of glucose concentration according to the invention and the horizontal axis showing the measurement values of glucose concentration according to the enzymatic electrode method. It is seen that a good correlation is obtained by measuring the oxygen supply and the blood flow volume according to the method of the invention (correlation coefficient=0.9324).

## Claims

1. A blood sugar level measurement apparatus comprising:

   a heat amount measurement portion for measuring a plurality of temperatures deriving from a body surface and obtaining information that is used for calculating the amount of convective heat transfer and the amount of radiation heat transfer, both of which are related to the dissipation of heat from said body surface;
   an oxygen amount measurement portion for obtaining information relating to the blood oxygen amount, said oxygen amount measurement portion comprising a blood flow volume measurement portion for obtaining information relating to the blood flow volume, and an optical measurement portion (18) for obtaining the hemoglobin concentration and the hemoglobin oxygen saturation in blood;
   an openable and closable cover (14) with which said optical measurement portion (18) can be covered;
   a cover open/close detection portion (57) for detecting whether said cover (14) is open or closed;
   a memory portion in which a relationship between parameters associated with said plurality of temperatures and said blood oxygen amount, and blood sugar levels is stored;
   a computation portion for converting a plurality of measurement values inputted from said heat amount measurement portion and said oxygen amount measurement portion into said parameters individually, and for computing a blood sugar level by applying said parameters to said relationship stored in said memory portion;
   a display portion (13) for displaying the result calculated by said computation portion; and
   an optical sensor correction portion (60) for correcting the output of said optical measurement portion (18), wherein:
   said blood flow volume measurement portion comprises a body-surface contact portion, a first temperature detector disposed adjacent to said body-surface contact portion, a second temperature detector for detecting the temperature at a location spaced apart from said body-surface contact portion, and a heat-conducting member connecting said body-surface contact portion and said second temperature detector, and wherein:

   said optical sensor correction portion (60) comprises an optical correction check portion (58) for calculating a correction value from the output of said optical measurement portion (18), and an optical correction portion (59) for correcting the output of said optical measurement portion (18) using the correction value calculated by said optical correction check portion.

2. The apparatus of claim 1, wherein said optical correction check portion (58) calculates said correction value by comparing a characteristics coefficient that is obtained on the basis of outputs of said optical measurement portion (18) when a light source is not emitting light and when it is emitting light, with an initial value that is measured and stored in advance.

3. The apparatus of claim 1, wherein said cover open/close detection portion (57) detects whether the cover (14) is open or closed by comparing an output of said optical measurement portion (18) when said light source is emitting light with a preset reference value.

4. The apparatus of claim 1, wherein said optical correction check portion (58) calculates a correction value from an output of said optical measurement portion (18) when said cover (14) is closed.

5. A blood sugar level measurement apparatus comprising:

   an ambient temperature measurement portion for measuring ambient temperature;
   a body-surface contact portion with which a body surface comes into contact;
   an adjacent temperature detector disposed adjacent to said body-surface contact portion;
   a radiation heat detector for measuring radiation heat from said body surface;
   a heat conducting member disposed adjacent to said body-surface contact portion;

an indirect temperature detector disposed in contact with said heat conducting member and at the same time spaced apart from said body-surface contact portion, for detecting the temperature at said location spaced apart from said body-surface contact portion;

an optical measurement portion (18) comprising a light source for irradiating said body-surface contact portion with light of at least two different wavelengths, and a photodetector;

an openable and closable cover (14) with which said optical measurement portion (18) can be covered;

a cover open/close detection portion (57) for detecting whether said cover (14) is open or closed;

a computation portion comprising a conversion portion and a processing portion, said conversion portion converting the outputs from said adjacent temperature detector, said indirect temperature detector, said ambient temperature measurement portion, said radiation heat detector, and said photodetector, into individual parameters, and said processing portion storing a relationship between said parameters and blood sugar levels in advance and calculating a blood sugar level by applying said parameters to said relationship;

an optical sensor correction portion (60) for correcting the output of said photodetector; and

a display portion (13) for displaying the result outputted from said computation portion, wherein:

said optical sensor correction portion (60) comprises an optical correction check portion (58) for calculating a correction value based on the output of said photodetector, and an optical correction portion (59) for correcting the output of said photodetector using the correction value calculated by said optical correction check portion.

6. The apparatus of claim 5, wherein said optical correction check portion (58) calculates said correction value by comparing a characteristics coefficient that is obtained on the basis of outputs of said photodetector when said light source is not emitting light and when it is emitting light, with an initial value that is measured and stored in advance.

7. The apparatus of claim 5, wherein said optical correction check portion (58) calculates a correction value based on the output of said photodetector when said cover (14) is closed.

8. The apparatus of claim 1 or 5, wherein said cover (14) comprises a reflector on the back surface thereof.

9. A blood sugar level measurement apparatus comprising:

an ambient temperature measurement portion for measuring ambient temperature;

a body-surface contact portion with which a body surface comes into contact;

an adjacent temperature detector disposed adjacent to said body-surface contact portion;

a radiation heat detector for measuring radiation heat from said body surface;

a heat conducting member disposed in contact with said body-surface contact portion;

an indirect temperature detector disposed adjacent to said heat conducting member and at the same time spaced apart from said body-surface contact portion, for detecting the temperature at said location spaced apart from said body-surface contact portion;

an optical measurement portion (18) comprising a light source for irradiating said body-surface contact portion with light of at least two different wavelengths, and a photodetector;

an openable and closable cover (14) comprising a reflector on the back surface thereof, said cover (14) enabling said optical measurement portion (18) to be covered, said reflector being used for the correction of said optical measurement portion (18);

a cover open/close detection portion (57) for detecting whether said cover (14) is open or closed;

a computation portion comprising a conversion portion and a processing portion, said conversion portion converting the outputs from said adjacent temperature detector, said indirect temperature detector, said ambient temperature measurement portion, said radiation heat detector, and said photodetector, into individual parameters, and said processing portion storing a relationship between said parameters and blood sugar levels in advance and calculating a blood sugar level by applying said parameters to said relationship; and

a display portion (13) for displaying the result outputted from said computation portion.

10. The apparatus of claim 5 or 9, wherein said cover open/close detection portion (57) detects whether said cover (14) is open or closed by comparing the output of said photodetector when said light source is emitting light with a preset reference value.

11. The apparatus of any of claims 1, 5 and 9, wherein said cover open/close detection portion (57) comprises a first switch (70) that is adapted to be depressed by said cover (14) when it is closed, and a second switch (20) that is adapted to be depressed by said cover (14) when it is open.

12. A blood sugar level measurement method comprising the steps of:

confirming that a measurement opening portion of an optical measurement portion (18) is closed by a cover (14) comprising a reflector disposed on the surface thereof, said optical measurement portion (18) comprising a light source for emitting light of a plurality of wavelengths and a photodetector;

obtaining a first measurement value by detecting the output of said photodetector without said light source emitting light;

obtaining a second measurement value by detecting the output of said photodetector when said light with said plurality of wavelengths is emitted in a sequential manner;

calculating a characteristics coefficient for each wavelength on the basis of said first and said second measurement values;

comparing said characteristics coefficient with an initial value that is measured and stored in advance, for each of said plurality of wavelengths, and then determining and storing a correction value if a fluctuation amount of said characteristics coefficient with respect to said initial value exceeds a preset threshold;

irradiating a test subject positioned at said measurement opening portion with the light of said plurality of wavelengths from said light source, and obtaining a third measurement value by detecting the output of said detector for each wavelength;

correcting said third measurement value for a corresponding wavelength using said correction value if said correction value is stored; and

determining information regarding hemoglobin concentration and information regarding hemoglobin oxygen saturation on the basis of said third measurement value that has been corrected by said correction value.

13. The method of claim 12, further comprising the step of, following the step of determining said correction value and storing it, determining whether said cover (14) is open or not, and, if closed, issuing an indication prompting that it be opened.

14. The method of claim 12, wherein the light of said plurality of wavelengths consists of wavelengths of 810 nm and 950 nm.

15. The method of claim 12, wherein said characteristics coefficient consists of the intercept and the slope of a line connecting said first measurement value and said second measurement value that are plotted in a coordinate system consisting of a horizontal axis showing diffuse reflectance and a vertical axis showing the output of said photodetector, with the diffuse reflectance of said reflector taken as 1.

16. The method of claim 15, wherein said fluctuation amount is evaluated in accordance with $(a(i)+b(i))/(a0(i)+b0(i))$, where a is the intercept and b is the slope that have been calculated with respect to the light of wavelength i, and a0 and b0 are their initial values, respectively.

17. The method of claim 16, wherein said correction value is determined in accordance with the following equations:

$$\text{Correction value G: } G(i) = b0(i)/b(i)$$

$$\text{Correction value O: } O(i) = a0(i) - b0(i) \times a(i)/b(i).$$

18. The method of claim 17, wherein a measurement value MM(i) after correction is determined from said third measurement value M(i) with respect to wavelength i in accordance with the following equation:

$$MM(i) = O(i) + G(i) \times M(i).$$

# FIG. 1

## FIG. 2

## FIG. 3

## FIG. 4

## FIG. 5

# FIG. 6

# FIG. 7

LCD *13*

BATTERY *41*

RAM *42*

IC CARD *43*

BUS LINE *44*

REAL-TIME CLOCK (RTC) *45*

MICROPROCESSOR

SOFTWARE-STORING ROM *56*

COVER OPEN/CLOSE DETECTION PORTION *57*

OPTICAL SENSOR CORRECTION PORTION *60*

OPTICAL CORRECTION CHECK PORTION *58*

OPTICAL CORRECTION PORTION *59*

*55*

ANALOG/DIGITAL CONVERTERS (AD1~AD5)

SENSOR PORTION *40*

PUSH-BUTTON SWITCHES

*11a*  *11b*  *11c*  *11d*

# FIG. 8

START

CIRCUIT TEST

DISPLAY PRODUCT NAME

11d

ERROR

OPTICAL CORRECTION CHECK

OPEN COVER          11d

COVER CLOSED

COVER OPEN/CLOSE CHECK

ERROR          COVER OPEN

PRE-MEASUREMENT

COVER OPEN &
CERTAIN TIME NOT
ELAPSED

CEVER CLOSED,
OR ERROR          0.1 SEC LATER

COVER OPEN/CLOSE CHECK

CERTAIN TIME ELAPSED IN PRE-
MESUREMENT

PLACE FINGER

MAIN MEASUREMENT: COUNTDOWN

CERTAIN TIME ELAPSED IN MAIN
MESUREMENT

LIFT FINGER

POST-MESUREMENT

COVER OPEN &
CERTAIN TIME NOT
ELAPSED

CEVER CLOSED,
OR ERROR          0.1 SEC LATER

COVER OPEN/CLOSE CHECK

CERTAIN TIME ELAPSED IN POST-
MESUREMENT

NO

OPTICAL CORRECTION
NECESSARY?

YES

OPTICAL CORRECTION

PROCESSING DATA

ERROR DISPLAY
& BUZZER          DISPLAY RESULT

11a

END

# FIG. 9

```
                                    ┌─────────────┐
                                    │    START    │
                                    └─────────────┘
                                           │
        ┌──────┐                           ▼
        │ 11d  │                  ┌─────────────────────────┐    ERROR
    ┌───┴──────────────┐          │  COVER OPEN/CLOSE CHECK  ├──────────┐
    │   CLOSE COVER     │          └─────────────────────────┘          │
    └───────────────────┘                 │                             │
          ▲     COVER OPEN      COVER CLOSED │                           │
          └──────────────────┐              ▼                           │
                             ┌─────────────────────────────────┐        │
                             │ OPTICAL-SYSTEM INTENSITY MEASUREMENT │     │
                             └─────────────────────────────────┘        │
                                           │                            │
                                           ▼                            │
                    ┌──────────────────────────────────────────────┐   │
                    │ OPTICAL-SYSTEM INTENSITY FLUCTUATION CALCULATION │ │
                    └──────────────────────────────────────────────┘   │
                                           │                            │
  CORRECTION NOT NECESSARY                 ▼               ERROR         │
  ┌────────────────────────┤    CORRECTION CHECK    ├──────────────┐    │
  │                         └─────────────────────┘                │    │
  │                                    │  CORRECTION NECESSARY       │    │
  │                                    ▼                             │    │
  │                         ┌─────────────────────┐                 │    │
  │                         │  CORRECTION VALUE SET │                 │    │
  │                         └─────────────────────┘                 │    │
  │                                    │                             │    │
  └────────────────────────────────────┤◄────────────────────────────────┘
                                        ▼
                                 ┌─────────────┐
                                 │     END     │
                                 └─────────────┘
```

# FIG. 10

# FIG. 11

# FIG. 12

| WAVELENGTH | DETECTOR | |
|---|---|---|
| | b | a |
| 810nm | 909.5032 | 9.119466 |
| 950nm | 467.6430 | 6.931648 |

# FIG. 13

| WAVELENGTH | DETECTOR | |
|---|---|---|
| | b0 | a0 |
| 810nm | 914.6332 | 9.444248 |
| 950nm | 477.4012 | 7.215467 |

# FIG. 14

y INTENSITY(mV)

$y=a0(i)+b0(i)*x$
(a0(i),b0(i): INITIAL
CHARACTERISTICS
VALUE OF INTENSITY)

SLOPE (b)

INTERCEPT (a)

0(NO LED
EMISSION: offset)

X DIFFUSE
REFLECTANCE(%)

1(MATERIAL)

# FIG. 15

| THRESHOLD | CORRECTION | PROCESS |
|---|---|---|
| 0.99≦FLUCTUATION AMOUNT (i)≦1.01 | NOT NECESSARY | NO CORRECTION |
| 1.01< FLUCTUATION AMOUNT (i)≦1.5<br>0.7≦ FLUCTUATION AMOUNT (i)<0.99 | NECESSARY | CORRECTION |
| 1.5< FLUCTUATION AMOUNT (i)<br>FLUCTUATION AMOUNT (i)<0.7 | NOT NECESSARY | ERROR |

FIG. 16

# FIG. 17

**European Patent Office**

## EUROPEAN SEARCH REPORT

Application Number

EP 04 03 0921

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | JP 03 566278 B1 (HITACHI, LTD) 15 September 2004 (2004-09-15) * the whole document * | 1-18 | A61B5/00 |
| E | -& EP 1 495 714 A (HITACHI, LTD) 12 January 2005 (2005-01-12) ----- | 1-18 | |
| A | PATENT ABSTRACTS OF JAPAN vol. 2000, no. 12, 3 January 2001 (2001-01-03) & JP 2000 258343 A (MITSUI MINING & SMELTING CO LTD; MITSUI & CO LTD), 22 September 2000 (2000-09-22) * abstract * ----- | 1,5,9 | |
| A | US 2003/010898 A1 (MACKENZIE HUGH ALEXANDER ET AL) 16 January 2003 (2003-01-16) * paragraph [0036] * * paragraph [0078]; figure 5 * ----- | 1,5 | |
| A | WO 01/28414 A (KAUFMANN-KIM, YUN-OAK; CHO, OK-KYUNG) 26 April 2001 (2001-04-26) * the whole document * ----- | 1,5,9 | TECHNICAL FIELDS SEARCHED (Int.Cl.7) A61B |

The present search report has been drawn up for all claims

| Place of search | Date of completion of the search | Examiner |
|---|---|---|
| Munich | 22 March 2005 | Alvazzi Delfrate, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 04 03 0921

This annex lists the patent family members relating to the patent documents cited in  the above-mentioned European search report.
The members are as contained in the European Patent Office EDP file on
The European Patent Office is in no way liable for these particulars which are merely  given for the purpose of information.

22-03-2005

| Patent document cited in search report | | Publication date | Patent family member(s) | | Publication date |
|---|---|---|---|---|---|
| JP 3566278 | B1 | 15-09-2004 | EP<br>US | 1495714 A1<br>2005010094 A1 | 12-01-2005<br>13-01-2005 |
| EP 1495714 | A | 12-01-2005 | JP<br>EP<br>US | 3566278 B1<br>1495714 A1<br>2005010094 A1 | 15-09-2004<br>12-01-2005<br>13-01-2005 |
| JP 2000258343 | A | 22-09-2000 | NONE | | |
| US 2003010898 | A1 | 16-01-2003 | AU<br>CA<br>EP<br>GB<br>GB<br>GB<br>WO<br>GB<br>JP<br>US | 6407998 A<br>2282855 A1<br>0967913 A1<br>2357844 A<br>2357845 A ,B<br>2357846 A ,B<br>9838904 A1<br>2322941 A ,B<br>2001526557 T<br>6403944 B1 | 22-09-1998<br>11-09-1998<br>05-01-2000<br>04-07-2001<br>04-07-2001<br>04-07-2001<br>11-09-1998<br>09-09-1998<br>18-12-2001<br>11-06-2002 |
| WO 0128414 | A | 26-04-2001 | WO | 0128414 A2 | 26-04-2001 |

EPO FORM P0459

For more details about this annex : see Official Journal of the European Patent Office, No. 12/82